# EUROPEAN PATENT SPECIFICATION

(11) **EP 1 249 265 B1**
(45) Date of publication and mention of the grant of the patent: **06.04.2005**
(21) Application number: 01129525.0
(22) Date of filing: 11.12.2001
(51) Int. Cl.: B01D 53/32, B03C 3/38, B01D 35/06, A61L 9/22

(54) **Air cleaner including a cold-plasma electrostatic catalytic device**
Vorrichtung zur Luftreinigung enthaltend ein Kaltplasma elektrostatisches katalytisches Gerät
Purificateur d'air comprenant un dispositif catalitique pour la generation d'un plasma froid electrostatique

(30) Priority: 13.04.2001 IT MI010816
(43) Date of publication of application: 16.10.2002
(73) Proprietor: DEPARIA ENGINEERING S.R.L., 23885 Calolziocorte (Lecco) (IT)
(72) Inventor: Vergani, Cristiano Carlo, 23885 Calolziocorte (Lecco) (IT)
(74) Representative: Cicogna, Franco

(56) References cited:
- EP-A- 1 086 740
- WO-A-99/12638
- WO-A-99/43419
- US-A- 5 582 632
- US-A- 5 746 051
- US-A- 5 843 288
- US-A- 5 914 015

## Description

### BACKGROUND OF THE INVENTION

The present invention relates to an air cleaner, including a cold-plasma electrostatic catalytic device.

As is known, a very important requirement in the air cleaning field, is that of providing an air cleaner specifically designed for removing from atmospheric air polluting substance particles.

Prior air cleaners conventionally provide to use filtering processes, exploiting electric, aerodynamic and mechanic force principles.

In particular. an air filtering using the gravity force principle would be very suitable for removing particles having a comparatively high weight.

In operation of such a gravity based device, the polluting particles are deposited in depositing chambers or plenum assemblies.

Further prior air cleaning or filtering processes provide to use water cyclones, in which water is used for increasing the weight of the suspended polluting particles.

Further dry cyclone filtering processes, on the other hand, provide to force a high speed air stream which, by impressing a rotary motion, causes said polluting substance particles to be separated under the centrifugal force effect.

A further filtering mechanism is the so-called "sieving" filtering, which operates to filter-out particles having a diameter greater than that of the fibrous net meshes constituting the filtering panel.

A further air filtering process is the so-called inertial or impact process, in which the air stream is abruptly deflected, whereas the polluting particles, under inertial forces, continue to move, to impact against the filter constructions, where they are deposited.

The lighter particles, as entrained by the air stream or flow, on the contrary, follow the contour or profile of the filter fibers.

However, if the movement trajectory of said lighter particles passes at a distance less than the particle ray, then, such a particle will adhere to the fiber under the effect of elementary electrostatic forces, or Van der Vaal's forces, according to the so-called shutoff or interception mechanism.

Particles having a further smaller diameter are held in the filters under the effect of a diffusion type of mechanism, according to which the particle is driven along a uneven trajectory, under the action of browniane molecular forces, so that said particle will end to adhere to a fibre, always under the effect of electrostatic forces.

Thus, starting from the understanding that, in most part of filtering mechanisms electrostatic forces are involved, further air cleaning filters have been designed, from the end of the XIX century to the present time, which are suitable to restrain polluting small particles, owing to the application of induced and ionizing electric fields.

Actually, said electrostatic filters have been ideally used in industrial environments, in which great amounts of gas or air must be processed, with a low power consume and high filtering yield.

Moreover, in recent years, said electrostatic filters have been also broadly used for cleaning air in indoor environments, either work and home environments, and this owing to their high efficiency in picking up the so-called "breathable fraction" of the atmospheric dust comprising particles having a diameter less than 6 µm, which can arrive up to the pulmonary deepest regions, with consequent very dangerous consequences.

In this connection it should be pointed out that a high cost and size of the above mentioned electrostatic filters has prevented their application in kitchen hoods, air conditioning systems, motor vehicle passenger compartments, or the like.

A great intrinsic drawback of the above disclosed technique, which drawback is accordingly substantially non-eliminable, is the requirement of additionally using adsorption grain panels, including, for example, active carbon grains or chemical reactive material impregnated grains, in order to provide a filtering action even on the non-particle polluting fractions.

Such a filtering stage addition would involve a very high cost and consume increase, and this also due to the great load losses introduced into the system.

Conventional electrostatic filters, notwithstanding their high efficiency, (actually they have a filtering efficiency equal to and greater than 99% according to the DOP Penetration Test standard), have the following drawbacks: an operating efficiency exclusively limited to the particles, a high cost, size and constructional complexity, as well as servicing and assembling difficulties.

A conventional electrostatic filter, as is known, comprises tens or hundreds or elements, which must be manually assembled by a skilled operator, which requires several labour hours with a great increase of the cost.

Furthermore, the servicing of such an electrostatic filter, which must be frequently and periodically performed, provides to wash the components by special washing machines including movable nozzles, or by ultrasound machines.

Gas treatment catalytic devices operating with a cold-plasma are known from WO-A-9943419 and US-A-5746051.

### SUMMARY OF THE INVENTION

Accordingly, the aim of the present invention is to overcome the above mentioned drawbacks, and, in particular, to provide an electrostatic filter having a performance equal to or greater than that of a conventional electrostatic filtering cell.

Within the scope of the above mentioned aim, a main object of the present invention is to provide such an air cleaner which has a high filtering efficiency in filtering the polluting fractions, comprising particle fractions and further polluting fractions of different natures.

Yet another object of invention is to provide such an electrostatic filter operating with a comparatively low power consume, and which can be assembled and serviced in a comparatively short time and which, moreover, is very reliable and safe in operation.

According to one aspect of the present invention, the above mentioned aim and objects, as well as yet other objects, which will become more apparent hereinafter, are achieved by an air cleaner which comprises an electrostatic catalytic device, operating based on the cold plasma properties.

### BRIEF DESCRIPTION OF THE DRAWINGS

Further characteristics and advantages of the present invention will become more apparent hereinafter from the following detailed disclosure of the preferred, though not exclusive, embodiment of an air cleaner including a cold-plasma electrostatic catalytic device, and which is illustrated, by way of an indicative, but not limitative, example in the figures of the accompanying drawings, where:

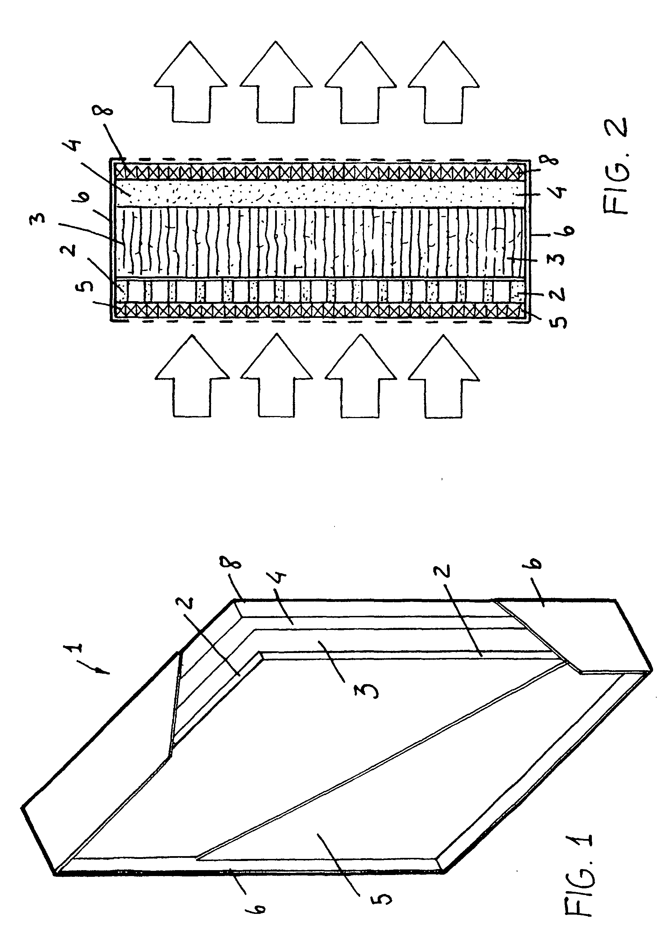

Figure 1 is a perspective view of an air cleaner according to the present invention;
Figure 2 is a cross-sectioned side view of the air cleaner according to the invention;

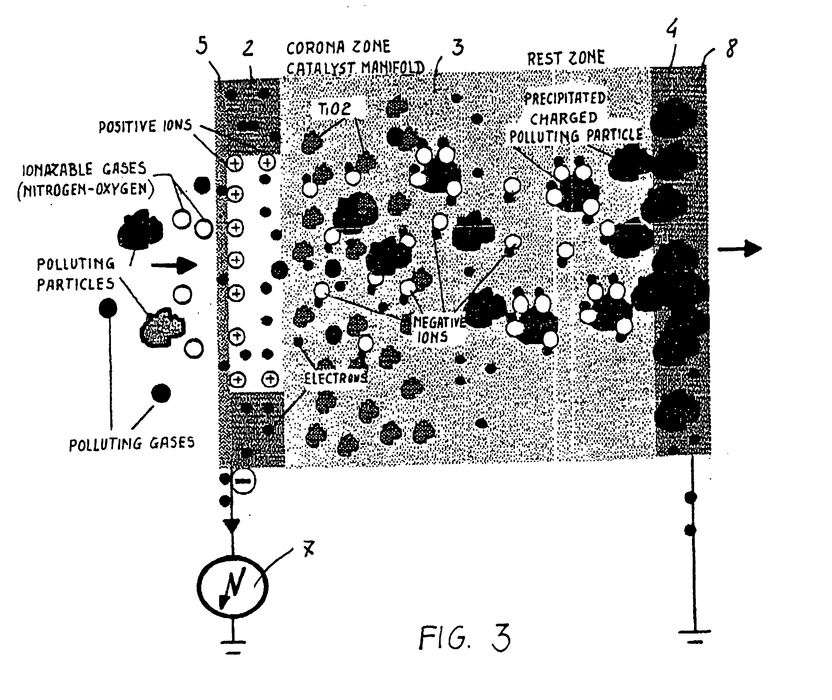

Figure 3 illustrates the operating principle of the electrostatic air cleaner according to the invention;
Figure 4 is a 3-D exploded view illustrating a modified embodiment of the air cleaner according to the present invention, including an interexchangeable cartridge.

### DESCRIPTION OF THE PREFERRED EMBODIMENTS

With reference to the number references of the above mentioned figures, the air cleaner according to the present invention, which has been generally indicated by the reference number 1, comprises a supporting framework, generally indicated by the reference number 6, including, through the overall depth thereof, a sandwich construction, comprising a metal net 5, coupled to a high voltage generator 7, with a polyester fiber perforated layer 2 loaded with active carbons, a polyester fiber layer 3, covered by a thin film of titanium dioxide (TiO₂), a polyester fiber layer 4, coated by an activated carbon coating, a metal net 8 coupled to the ground of said high voltage generator 7.

The elements 5 and 2 form an electrode of a device for generating a strong flow of free electrons, unstable ions and molecules, of a strongly reactive type.

The above mentioned flow is called "cold plasma" or non-thermal plasma, for distinguishing it from a thermal status plasma in which the molecular ionization is induced by the thermal energy produced by a temperature increase.

The non thermal or cold plasma, on the contrary, is generated by an electromagnetic energy, i.e. by applying a high pulsed potential difference with respect to a reference electrode comprising the stages 4 and 8 and being electrically coupled to the ground.

Said potential difference will provide, by induction, an electric field having a corresponding amplitude or strength and an opposite orientation, which operates to perform an ionizing process.

The mentioned layer 3 is traversed by the above disclosed flow, and, being arranged between the two mentioned electrodes, it will have a double function of collector or manifold and of catalytic reactor.

The polluting particle fraction, being suspended in air, as it is subjected to said cold plasma, will assume an electrostatic charge allowing it to be attracted by the polyester fibers of the layer 3, having an opposite-sign electrostatic charge.

Depending on the acquired charge amount, which, in turn, depends on the physical-geometrical and chemical characteristics of the individual particles, the latter will be attracted by the fibers of the layer 3 or the fibers of the following layer 4.

Then, in their trajectory path, corresponding to the layer 3, the volatile organic substances are subjected to an oxidation process.

In fact, the cold plasma is characterized by the presence of greatly accelerated and energetic electrons, which, by impacting against the environment-temperature air molecules, will form oxidating radicals OH⁻ and O⁻, which can directly react with the polluting substances to form other oxidating substances such as O₃, OOH, O₂-.

Moreover, a great photon amounts having a wave length in the UV spectrum range from 160 to 220 nm, and providing two important effects, are generated.

The first of said effect is the actuating of the photocatalytic properties of said titanium dioxide, due to the passage of the electrons from the valence band to the conductive band, for which it is sufficient the energy of a photon of 3.2 eV, that is at a wave length less than 387 nm.

In particular, the photons generated inside the plasma are suitable to activate or energize said titanium dioxide, thereby forming, after a series of reactions involving charge transfers, further free oxidating radicals.

A direct consequence of this is that the polluting molecules, which are passing through an environment saturated by strongly oxidating chemical species, are quickly decomposed.

The second effect is that of a strong biocide action, due to the UV radiation (160-220 nm), to which the most part of pathogen microorganisms are greatly sensible, owing to the irreversible and noxious alterations produced by the UV radiation in the intracellular structure; for example, a formation of free radical in cytoplasm.

The layer 4, having a low electric resistivity, mainly operates as a reference electrode for the electric field generating the cold plasma.

Moreover, said layer 4 operates as a manifold for collecting the electrostatically charged particle present in the preceding sections of the air cleaner.

The known absorbency characteristics of the gaseous phase polluting substances are supplied to the layer 4 by the presence of the activated carbon, contributing to a neutralization of the gaseous phase polluting substances.

The above disclosed air cleaner can be made according to several modified embodiments.

For a civil and industrial environment use, the fibrous layer sandwich can be held in a flexible construction, to better fit the volumes of existing apparatus, for which the space parameter is very critical, for example in the motor vehicle and aerospace field.

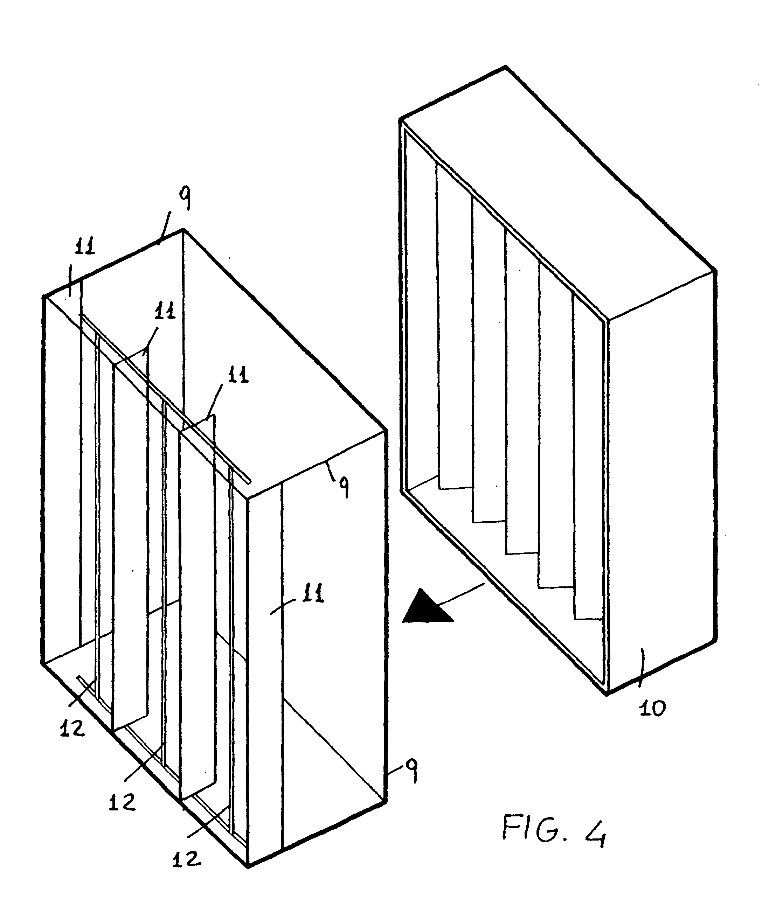

In the embodiment shown in figure 4, the fibrous layer sandwich construction constitutes a cartridge which can be easily applied and replaced in the form of a cartridge 10 which is introduced into a metal frame 9.

The metal frame 9, which is also called "caddy", having a shape and outer size identical to those of the above mentioned framework 6 according to the invention, comprises, on the air inlet front side thereof, an ionization electrostatic barrier, formed by a grid of equispaced electrodes 11, coupled to the ground, and by a further grid, intertwined with the preceding grid, of tungsten or carbon equispaced wires 12, coupled to the high voltage generated by the high voltage generator 7.

The remaining portion of the volume on the back of the metal framework 9 is provided for housing therein the mentioned interexchangeable or replaceable cartridge 10.

This modified embodiment provides the same broad spectrum filtering operating characteristics, i.e.: particles, chemical substances, polluting gases and yet other substances.

This modified embodiment, however, allows, at the end of the operating life of the filter, instead of disposing of the overall device, to replace only the interexchangeable cartridge 10, thereby reconstructing the functional structure of the air cleaner, with a full replacing of only the manifold, where the removed polluting residue are collected.

In this connection it should be pointed out that the subject air cleaner device is characterized in that it comprises a corona-effect ionization barrier, which constitutes a portion of an assembly cooperating for forming a room-temperature plasma generator.

This device substantially comprises a metal net, or a net made of any other suitable electroconductive material, coupled to a specifically designed high voltage generator, contacting a following electroconductive fibrous material layer, comprising, for example, polyester fibers coated by activated carbon, metal fibers, carbon fibers and the like.

One or more openings have been formed through the mentioned fibrous material layer, which openings can have any desired shape and size: the area not involved by said openings can either allow or not the passage of air by applying a plastics material film or other suitable material film.

The subject device comprises, moreover, a manifold including a loose fibrous or granular material layer, restrained by a restraining frame or having a sponge like construction, not electrically conductive, made of a flat or pleated plate, added with titanium dioxide (TiO₂), of an Anatase or Rutile type, or any suitable mixture of these two types, with an optional addition of substances adapted to extend the wave length range of the electromagnetic radiation necessary for activating said TiO₂, such as Ru compounds and the like.

The subject device comprises furthermore a second manifold, constituting a portion of an assembly forming a room temperature plasma generator, including a loose fibrous or granular material layer, restrained by a restraining frame, or having a textured construction, for example an extruder construction, or a sponge like construction, electrically conductive, with an optional addition of TiO₂ under a flat plate or pleated configuration, contacting a metal net, or any desired net made of a suitable electroconductive material, coupled to the earth pole of a suitable high voltage generator.

The air cleaner device according to the present invention comprises, between its two component parts, a loose fibrous or granular material layer, restrained by a restraining frame, or having a textured construction (for example an extruded construction), or an electrically conductive sponge like construction.

Said device can also comprise active carbon coated polyester fibers, metal fibers, carbon fibers and the like, either in a flat plate or in a pleated configuration.

The present air cleaner device comprises, in other words, a construction specifically designed for generating room environment or cold plasma, including one or more windings of electroconductive material wound about dielectric material ducts, through the inside of which air directed to following construction flows.

Said windings are coupled to a suitable RF generator, to generate said cold plasma in said gaseous or air substance.

The air cleaner device comprises, moreover, in particular, a construction including a dielectric material framework, supporting a pattern of electroconductive material wires (for example made of tungsten or carbon), equispaced from one another and coupled to a suitable high voltage generator, therebetween can be intertwined earth coupled conductive material plates.

The construction of the subject device can be further provided with a metal framework, which, in turn, can receive the structures held in an inter-exchageable box element, operating as a manifold element.

Thus, at the end of the device operating life, said box element can be removed and replaced by a new one, thereby recovering the full operating capabilities of the air cleaning device.

Said metal framework is characterized by a shape, a size and an electric contact arrangement suitable to allow to easily replace the commercially available electrostatic filters in finished apparatus, without requiring any modifications of the supporting structures.

Moreover, it is provided to deposit, on the air exposed gaseous substance structures, a titanium dioxide (TiO₂) film of an Anatase or Rutile type, or in a mixture according to any desired mixing ratio, with an optional addition of substances suitable to extend the wave length range of the electromagnetic radiation necessary to activate said TiO₂, such as Ru and the like compounds, according to the following method:
a) preparing of a 15% solution of polyvinyl alcohol in water; immersing the particle material to be coated into this solution and then drying to room temperature;
b) providing a 15% suspension of TiO₂ in water;
c) immersing the particle material to be coated into said suspension and percolating the excess material;
d) quickly drying the particle material through the exposition to a RF field having a frequency from 2 to 4 GHz for few seconds.

The above method allows to make a firm coating, of a highly reactive nature, since it does not include polluting substances, and this without damaging the supporting material even if the latter comprises a material having a poor resistance against high temperature and organic solvents, such as polyester fibers.

From the above disclosure it should be apparent that the invention fully achieves the intended aim and objects.

In particular, the fact is to be pointed out that an electrostatic air cleaner has been provided, characterized by the use of a cold plasma barrier, i.e. at room temperature, specifically designed for providing an optimum broad spectrum filtering of the polluting substances.

This air cleaner has a very high filtering efficiency, very low making and servicing costs, and a very small power drain.

## Claims

1. An air cleaner including a cold-plasma electrostatic catalytic device, **characterized in that** said air cleaner comprises a supporting framework (6) and a sandwich construction, including a first metal net (5) coupled to a high voltage generator (7), a first perforated layer of polyester fibers (2), loaded by activated carbons, a second polyester fiber layer (3) coated by a thin film of titanium dioxide, a third polyester fiber layer (4) coated by activated carbon, and a second metal net (8) coupled to the ground of said high voltage generator (7).

2. An air cleaner, according to claim 1, **characterized in that** said metal net (5) and polyester fiber layer (2) provide a first electrode of said cold-plasma electrostatic catalytic device.

3. An air cleaner, according to claim 2, **characterized in that** said third polyester fiber layer (4) and said second metal net (8) provide a second reference electrode electrically coupled to said ground of said high voltage generator (7).

4. An air cleaner, according to claim 3, **characterized in that** said second polyester fiber layer (3) is intertwined between said first and second electrodes (5, 2; 4, 8).

5. An air cleaner, according to claim 1, **characterized in that** said air cleaner has an outer flexible construction.

6. An air cleaner, according to claim 1, **characterized in that** said air cleaner comprises a metal framework (9) having the same size and shape as said supporting framework (6) and including, at an air inlet front-side thereof, an ionizing electrostatic barrier formed by a first electrode grid (11), having electrodes coupled to said ground, and a second electrode grid, spatially intertwined with said first electrode grid, including tungsten or carbon wires (12) coupled to said high voltage generator (7).

7. An air cleaner, according to claim 6, **characterized in that** said metal framework (9) houses an interexchangeable cartridge (10) having said sandwich construction.

8. An air cleaner, according to claim 1, **characterized in that** said air cleaner further comprises a room temperature plasma generator, at least a first manifold formed of a loose fibrous material layer restrained by a first-manifold restraining frame or textured or in a sponge form, electrically non conductive, either in a flat plate or pleated configuration, added with dioxide titanium of an anatase or rutile type or a mixture thereof, with an optional addition of Ru compounds, and a second manifold constituting a portion of said cold plasma generator and comprising a loose fibrous layer restrained by a second-manifold restraining frame or textured or in an electrically conductive sponge form, added with titanium dioxide either in a flat plate or pleated configuration, contacting a metal or other electrically conductive material net, coupled to said ground of said high voltage generator.

9. An air cleaning device, according to claim 8, **characterized in that** said air cleaning device comprises two of said first manifolds therebetween being arranged said loose fibrous layer.

10. An air cleaning device, according to claim 8, **characterized in that** said room temperature plasma generator comprises one or more electroconductive material windings wound about dielectric material ducts, through which a gas or air flows, said windings being coupled to a RF generator, to form plasma in said gas or air.

11. An air cleaning device, according to claims 8 to 10, **characterized in that** said plasma generator is arranged in a metal frame which also houses said first and second manifolds.

## Patentansprüche

1. Luftfilter einschließlich elektrostatischer katalytischer Kaltplasma-Vorrichtung, **dadurch gekennzeichnet, dass** der Luftfilter einen unterstützenden Rahmen (6) und eine Sandwich-Konstruktion einschließlich einem ersten Metallnetz (5), das an einem Starkstrom-Erzeuger (7) angeschlossen ist, eine erste perforierte Schicht aus Polyesterfasern (2), die mit Aktivkohle angereichert ist, eine zweite mit einem dünnen Titandioxid-Film überzogene Polyesterfaser-Schicht (3), eine dritte mit Aktivkohle überzogene Polyesterfaser-Schicht (4), und ein zweites Metallnetz (8), welches am Starkstrom-Erzeuger (7) angeschlossen ist, enthält.

2. Luftfilter nach Anspruch 1, **dadurch gekennzeichnet, dass** das Metallnetz (5) und die Polyesterfaser-Schicht (2) eine erste Elektrode der elektrostatischen katalytischen Kaltplasma-Vorrichtung bereitstellen.

3. Luftfilter nach Anspruch 2, **dadurch gekennzeichnet, dass** die dritte Polyesterfaser-Schicht (4) und das zweite Metallnetz (8) eine zweite Referenzelektrode bereitstellen, die elektrisch am Starkstrom-Erzeuger (7) angeschlossen ist.

4. Luftfilter nach Anspruch 3, **dadurch gekennzeichnet, dass** die zweite Polyesterfaser-Schicht (3) mit den ersten und zweiten Elektroden (5, 2; 4, 8) verflochten ist.

5. Luftfilter nach Anspruch 1, **dadurch gekennzeichnet, dass** der Luftfilter eine flexible Außenkonstruktion aufweist.

6. Luftfilter nach Anspruch 1, **dadurch gekennzeichnet, dass** der Luftfilter einen Metallrahmen (9) der gleichen Größe und Form aufweist wie der unterstützende Rahmen (6), einschließlich einer ionisierenden elektrostatischen Schranke an einem Lufteinlass an dessen Vorderseite, die durch ein mit Elektroden am Bezugspotential verbundenes erstes Elektrodenraster (11) geformt ist, und ein zweites Elektrodenraster, das räumlich mit dem ersten Elektrodenraster verbunden ist, einschließlich Wolfram- oder Kohledrähten (12) die am Starkstrom-Erzeuger (7) angeschlossen sind.

7. Luftfilter nach Anspruch 6, **dadurch gekennzeichnet, dass** der Metallrahmen (9) ein austauschbares Steckmodul (10) beherbergt, das die Sandwich-Konstruktion aufweist.

8. Luftfilter nach Anspruch 1, **dadurch gekennzeichnet, dass** der Luftfilter weiter einen Raumtemperatur-Plasma-Erzeuger enthält, wenigstens einen ersten Verteiler aus einer losen faserigen Materialschicht, die durch einen Erstverteiler-Rahmen zurückgehalten wird oder die strukturiert oder in einer elektrisch nicht leitenden Schwammform ist, entweder in einer flachen Schale oder in gefalteter Art angeordnet, mit Titandioxid aus einer Art Anatas oder Rutil oder einer Mischung hiervon versetzt, mit der Möglichkeit Ru-Verbindungen beizufügen, und einen zweiten Verteiler, der einen Abschnitt des Kaltplasma-Erzeugers bildet und eine lose faserige Schicht enthält, welche durch einen Zweitverteiler-Rückhalterahmen zurückgehalten wird oder die strukturiert oder in einer elektrisch nicht leitenden Schwammform ist, mit Titandioxid versetzt, entweder in einer flachen Schale oder gefalteten Art angeordnet, ein Netz aus Metall oder anderweitig elektrisch leitendem Material berührend und mit dem Bezugspotential des Starkstrom-Erzeugers verbunden.

9. Luftfilter nach Anspruch 8, **dadurch gekennzeichnet, dass** der Luftfilter zwei der ersten Verteiler enthält, die zwischen der losen faserigen Schicht angeordnet sind.

10. Luftfilter nach Anspruch 8, **dadurch gekennzeichnet, dass** der Raumtemperatur-Plasma-Erzeuger ein oder mehrere elektrisch leitende Materialwicklungen enthält, die um Kanäle aus dielektrischem Material gewickelt sind, durch die Gas oder Luft strömt, wobei die Wicklungen an einem HF-Erzeuger angeschlossen sind um im Gas oder in der Luft Plasma zu erzeugen.

11. Luftfilter nach Ansprüchen 8 bis 10, **dadurch gekennzeichnet, dass** der Plasma-Erzeuger in einem Metallrahmen angeordnet ist, der ebenfalls die ersten und zweiten Verteiler beherbergt.

## Revendications

1. Un purificateur d'air comprenant un dispositif catalytique électrostatique à plasma froid, **caractérisé en ce que** ledit purificateur d'air comprend un cadre support (6) et une structure en sandwich, comprenant un premier maillage métallique (5) relié à un générateur de haute tension (7), une première couche perforée de fibres de polyester (2), chargée de charbons actifs, une seconde couche de fibres de polyester (3) revêtue d'un mince film de dioxyde de titane, une troisième couche de fibres de polyester (4) revêtue de charbon actif, et un second maillage métallique (8) relié à la masse dudit générateur de haute tension (7).

2. Un purificateur d'air selon la revendication 1, **caractérisé en ce que** ledit maillage métallique (5) et ladite couche de fibres de polyester (2) constituent une première électrode dudit dispositif catalytique électrostatique à plasma froid.

3. Un purificateur d'air selon la revendication 2, **caractérisé en ce que** ladite troisième couche de fibres de polyester (4) et ledit second maillage métallique (8) constituent une seconde électrode de référence reliée électriquement à ladite masse dudit générateur de haute tension (7).

4. Un purificateur d'air selon la revendication 3, **caractérisé en ce que** ladite seconde couche de fibres de polyester (3) est entrelacée entre lesdites première et seconde électrodes (5, 2 ; 4, 8).

5. Un purificateur d'air selon la revendication 1, **caractérisé en ce que** ledit purificateur d'air présente une structure extérieure souple.

6. Un purificateur d'air selon la revendication 1, **caractérisé en ce que** ledit purificateur d'air comprend un cadre métallique (9) ayant les mêmes dimension et forme que ledit cadre de support (6) et comprenant, sur l'un de ses côtés frontaux d'entrée d'air, une barrière électrostatique ionisante formée par une première électrode en grille (11), ayant des électrodes reliées à ladite masse, et une seconde électrode en grille, entrelacée spatialement avec ladite première électrode en grille, comprenant des fils en tungstène ou carbone (12) reliés audit générateur de haute tension (7).

7. Un purificateur d'air selon la revendication 6, **caractérisé en ce que** ledit cadre métallique (9) loge une cartouche interchangeable (10) comportant ladite structure en sandwich.

8. Un purificateur d'air selon la revendication 1, **caractérisé en ce que** ledit purificateur d'air comprend en outre un générateur de plasma à température ambiante, au moins un premier collecteur formé d'une couche de matériau fibreux sans cohésion encastrée dans un premier cadre de maintien de premier collecteur ou texturée ou sous forme spongieuse, non électriquement conductrice, sous une configuration de plaque plane ou plissée, additionnée de dioxyde de titane de type anatase ou rutile ou d'un de leurs mélanges, avec une addition éventuelle de composés du Ru, et un second collecteur constituant une partie dudit générateur de plasma froid et comprenant une couche de fibres sans cohésion encastrée dans un cadre de maintien de second collecteur ou texturée ou sous forme d'éponge conductrice électriquement conductrice, additionnée de dioxyde de titane, sous une configuration de plaque plane ou plissée, en contact avec un maillage de métal ou d'un autre matériau électriquement conducteur, relié à ladite masse dudit générateur de haute tension.

9. Un purificateur d'air selon la revendication 8, **caractérisé en ce que** ledit purificateur d'air comprend deux desdits premiers collecteurs, ladite couche de fibres sans cohésion étant disposée entre eux.

10. Un purificateur d'air selon la revendication 8, **caractérisé en ce que** ledit générateur de plasma à température ambiante comprend un ou plusieurs enroulements de matériau électriquement conducteur enroulés autour de conduits en matériau diélectrique, au travers desquels un gaz ou de l'air circule, lesdits enroulements étant reliés à un générateur RF, pour former un plasma dans ledit gaz ou air.

11. Un purificateur d'air selon les revendications 8 à 10, **caractérisé en ce que** ledit générateur de plasma est disposé dans un cadre métallique qui loge aussi lesdits premier et second collecteurs.
